# FASCICULE DE BREVET EUROPEEN

(11) **EP 2 640 310 B1**
(45) Date de publication et mention de la délivrance du brevet: **12.11.2014**
(21) Numéro de dépôt: 11782173.6
(22) Date de dépôt: 16.11.2011
(51) Int. Cl.: A61D 7/04, A01K 1/03, G01N 33/50, G01N 33/497

(54) **Dispositif d'analyse des effets d'au moins un paramètre relatif à une substance volatile**
Vorrichtung zur Analyse der Auswirkungen von mindestens einem Parameter bezüglich eines flüchtigen Stoffes
Device for analyzing the effects of at least one parameter relating to a volatile substance

(30) Priorité: 16.11.2010 FR 1059417
(43) Date de publication de la demande: 25.09.2013
(73) Titulaire: Immunosearch, 06130 Le Plan De Grasse (FR)
(72) Inventeur: STOIAN, Alina Valentina, F-34090 Montpellier (FR); SANCHEZ, José, Gregorio, F-34160 Sussargues (FR); DRUON, Stéphanie, F-34725 Jonquieres (FR); GROUX, Hervé, F-06650 Le Rouret (FR)
(74) Mandataire: Regimbeau
(86) Numéro de dépôt international: PCT/EP2011/070287
(87) Numéro de publication internationale: WO 2012/066055

(56) Documents cités:
- WO-A1-01/77364
- DE-U1- 20 304 054
- FR-A1- 2 849 057
- US-A- 5 379 777
- US-A- 5 928 860
- US-A1- 2009 211 534
- US-B1- 7 195 899

## Description

L'invention concerne le domaine de l'analyse de la toxicité de produits pour des êtres vivants (humains, animaux, environnement aquatique ou aérien).

Généralement, ces analyses se font en utilisant des systèmes d'essai vivants, qui peuvent aussi bien être des animaux aériens ou aquatiques, des plantes, des algues, ou encore des cultures de cellules animales, végétales ou bactériennes. L'ensemble de ces systèmes d'essai respire et vit par échange d'oxygène et de gaz carbonique de l'air et ne peut donc pas survivre dans des enceintes hermétiquement closes pendant de longues périodes.

Quelles que soient les procédures envisagées dans des problématiques aussi différentes que l'irritation, la sensibilisation ou l'écotoxicité, on réalise généralement différents tests in vitro mettant en présence ces systèmes d'essai biologiques avec des substances chimiques naturelles ou synthétiques dont on veut évaluer des paramètres d'intérêt tels que la toxicité. On évalue alors une série de perturbations métaboliques, par exemple la variation de l'expression de biomarqueurs de l'activité biologique des systèmes d'essai, leur mortalité et/ou leurs modifications métaboliques.

Néanmoins, lorsque les substances à tester sont volatiles, leur concentration varie dans le temps, notamment en fonction de leur volatilité et du lieu où les analyses sont réalisées. Il est donc difficile de connaître à tout instant la quantité réelle de substance en contact avec le système d'essai. Pour cela, l'état de l'art privilégie généralement deux solutions.

La première consiste à réaliser les analyses dans des enceintes fermées afin de maintenir la concentration de la substance volatile constante. Néanmoins, cette solution présente l'inconvénient de réduire la durée possible de l'analyse, sous peine de tuer les systèmes d'essai et les privant d'oxygène.

La deuxième solution consiste à réaliser les essais dans de larges enceintes, voire des enceintes ouvertes afin de garantir la présence de suffisamment d'air pour le système d'essai au cours de l'expérience. Il est alors possible de réaliser des essais sur de plus longues durées. Cependant, l'évaporation de la substance volatile implique des variations de la concentration qui ne sont pas maîtrisées et influent sur les résultats.

Une autre possibilité est de mettre en oeuvre des chimiothèques répertoriant un certain nombre de molécules volatiles plus ou moins solubles dans différents solvants. De telles chimiothèques, qui répertorient en particulier des molécules qui présentent un potentiel sensibilisant ou irritant ou susceptibles d'amener des perturbations de la faune et la flore environnementales, sont par exemple mises en oeuvre dans le domaine des parfums et des cosmétiques. Ces chimiothèques ne sont cependant pas exhaustives et ne peuvent remplacer des analyses sur des systèmes d'essai.

Le document US 5 928 860 décrit un procédé de détermination de la tolérance de l'organisme humain ou animal vis-à-vis de la toxicité de substances gazeuses, liquides et/ou visqueuses. Un échantillon de tissu est placé à cet effet dans une chambre qui comprend au moins deux compartiments séparés l'un de l'autre par l'échantillon de tissu. Une solution comprenant une substance nutritive est fournie de part et d'autre du tissu, et le tissu est soumis à une substance test au niveau d'un de ces côtés. Le tissu est alors analysé pour déterminer la toxicité de la substance.

Un objectif de l'invention est donc de proposer un procédé et un dispositif permettant d'analyser de manière fiable et reproductible certains paramètres de substances chimiques, en particulier des substances volatiles, sur des systèmes d'essai, en maîtrisant la concentration de la substance tout en permettant aux systèmes d'essai de respirer tout au long de l'analyse dans des conditions d'essais pouvant être stériles, les paramètres pouvant notamment être la toxicité des substances volatiles, leur fonctionnalité, leur potentiel allergène, leur potentiel mutagène, leur potentiel de reprotoxicité, leur toxicité pour un milieu aquatique, leur potentiel de biodégradation, leur possibilité de transformation en d'autres composés par réaction de polymérisation, d'oxydation ou encore de réduction, leurs effets pharmacologiques (notamment par une analyse d'interaction entre le composé volatile et le système d'essai pendant un intervalle de temps déterminé et avec une concentration déterminée généralement plus grande que celles permises par l'état de l'art actuel), etc.

De manière secondaire, un autre objectif de l'invention est de proposer un procédé permettant de calibrer le dispositif d'analyse, afin de garantir l'exactitude des résultats de l'analyse.

Pour cela, l'invention propose un dispositif d'analyse des effets d'au moins un paramètre relatif à une substance volatile sur un système d'essai vivant, caractérisé en ce qu'il comprend :
- une enceinte composée d'un premier compartiment comprenant une entrée et une sortie de gaz, et d'un deuxième compartiment dans lequel sont disposés le système d'essai et la substance volatile,
- les premier et deuxième compartiments communiquant l'un avec l'autre par l'intermédiaire d'une ouverture munie d'une membrane hémiperméable adaptée pour limiter de manière déterminée un flux de substance volatile du deuxième compartiment vers le premier compartiment, tout en permettant un flux de gaz du premier compartiment vers le deuxième compartiment, et
- le deuxième compartiment étant hermétique de sorte que les seuls échanges avec un milieu extérieur aient lieu vers le premier compartiment, à travers la membrane.

Certains aspects préférés mais non limitatifs du dispositif d'analyse selon l'invention sont les suivants :
- le paramètre est la toxicité de la substance volatile ;
- le gaz comprend 5% de dioxyde de carbone et 95% d'humidité relative ;
- la substance volatile est mélangée à un fluide de manière à obtenir une solution mère, et le dispositif comprend en outre une zone adaptée pour recevoir ladite solution mère ;
- le fluide utilisé dans l'obtention de la solution mère est de l'eau distillée ou de l'eau extra pure ;
- la solution mère est introduite par l'intermédiaire d'une vanne ; et
- la membrane est fixée sur l'ouverture par l'intermédiaire d'un joint.

Selon un deuxième aspect, l'invention propose un procédé d'analyse des effets d'au moins un paramètre relatif à une substance volatile sur un système d'essai vivant à l'aide d'un dispositif conforme à l'invention, caractérisé en ce qu'il comprend les étapes suivantes :
- mélanger la substance volatile avec un fluide de manière à obtenir une solution mère homogène,
- placer la membrane sur l'ouverture de manière étanche,
- introduire la solution mère dans le deuxième compartiment et fermer le deuxième compartiment, de sorte que les seuls échanges avec un milieu extérieur aient lieu vers le premier compartiment, à travers la membrane,
- faire passer un flux gazeux dans le premier compartiment pendant un temps déterminé,
- déterminer l'évolution dans le temps de la concentration de la substance volatile dans le deuxième compartiment, et
- à partir de l'évolution dans le temps de la concentration de la substance volatile, analyser le système d'essai et en déduire le paramètre relatif à la substance volatile.

Certains aspects préférés mais non limitatifs du procédé d'analyse sont les suivants :
- il comprend en outre une étape au cours de laquelle l'enceinte est placée à une température déterminée préalablement à l'introduction de la solution mère ;
- la solution mère et le flux gazeux balayant le premier compartiment sont introduits à la température déterminée de l'enceinte ;
- la température déterminée est d'environ 37°C +/- 1°C ;
- l'évolution dans le temps de la concentration de la substance volatile est déterminée en fonction de l'un au moins des paramètres suivants :
- la surface de transfert de la solution mère dans le deuxième compartiment,
- le volume du deuxième compartiment,
- la hauteur de la solution mère dans le deuxième compartiment,
- le volume de solution mère et/ou le volume de gaz présent dans le deuxième compartiment,
- la température de la solution mère ou du gaz présent dans le deuxième compartiment,
- la pression dans le premier compartiment et/ou dans le deuxième compartiment,
- la composition du gaz balayant le premier compartiment,
- le débit de gaz dans le premier compartiment,
- des propriétés de la substance volatile et,
- des propriétés de la membrane ;
- les propriétés de la substance volatile comprennent l'un au moins des éléments du groupe suivant : son coefficient de partage, son coefficient de diffusion dans l'eau, son coefficient de diffusion dans l'air, sa viscosité en phase liquide, sa viscosité en phase vapeur, sa densité en phase liquide, et sa densité en phase vapeur ;
- les propriétés de la membrane comprennent l'un au moins de éléments du groupe suivant : sa porosité, la taille de ses pores, et sa tortuosité ; et
- il comprend en outre une étape préalable d'homogénéisation de la solution mère.

D'autres caractéristiques, buts et avantages de la présente invention apparaîtront mieux à la lecture de la description détaillée qui va suivre et en regard des dessins annexés donnés à titre d'exemples illustratifs et sur lesquels :
La figure 1 représente une vue en coupe d'une forme de réalisation d'un dispositif conforme à l'invention,
La figure 2 représente un exemple d'installation du dispositif au cours de l'analyse, et
Les figures 3a, 3b et 3c représentent des vues de face et en coupe de l'élément de séparation des deux compartiments, du premier compartiment et du deuxième compartiment respectivement.

Nous allons à présent décrire un dispositif d'analyse des effets d'au moins un paramètre relatif à une substance sur un système d'essai vivant conforme à l'invention.

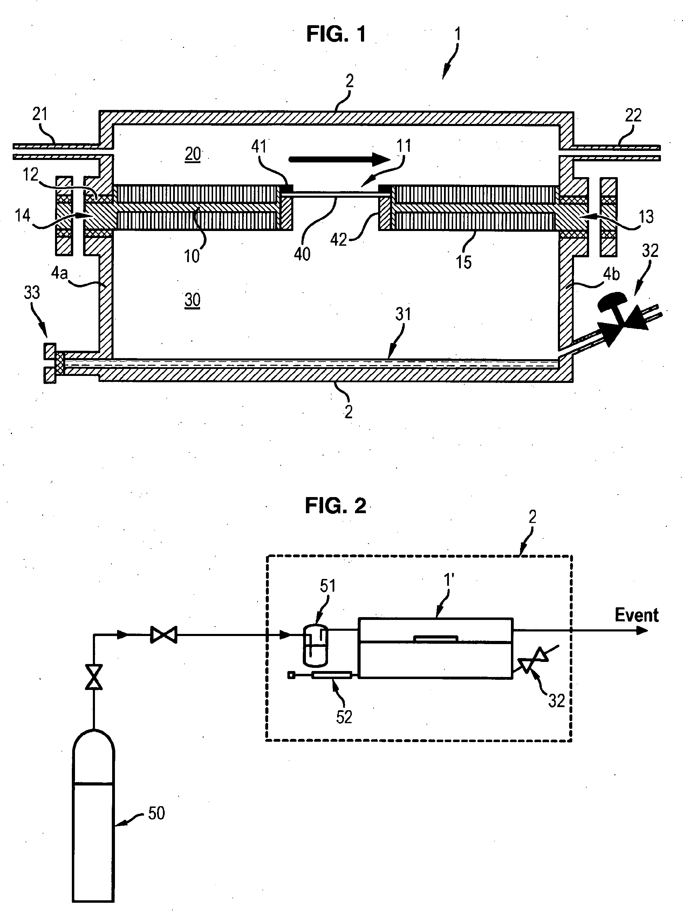

En relation avec la figure 1, on a représenté une forme de réalisation d'un dispositif. Le dispositif comprend une enceinte 1, de préférence, comprenant une paroi supérieure 2 et une paroi inférieure 3 reliées entre elles de manière étanche par quatre parois latérales 4a, 4b, 4c, 4d. Les parois 2-4d peuvent notamment être réalisées en acier inoxydable.

L'enceinte 1 comporte en outre une paroi interne de séparation 10 qui s'étend entre deux parois latérales opposées de l'enceinte de manière à définir deux compartiments 20, 30.

Ici, un premier compartiment 20 s'étend en partie supérieure de l'enceinte 1, entre la paroi supérieure 2 et la paroi de séparation, tandis que le deuxième compartiment 30 s'étend entre la paroi de séparation 10 et la paroi inférieure 3. Ceci n'est cependant pas limitatif : la paroi de séparation 10 peut en effet s'étendre entre la paroi supérieure et la paroi inférieure de manière à séparer verticalement les deux compartiments 20 et 30.

La paroi de séparation 10 est de préférence pleine, à l'exception d'une ouverture 11 de dimensions déterminées, permettant aux compartiments 10 et 20 de communiquer entre eux.

Les dimensions de l'ouverture 11 dépendent des conditions d'analyse et peuvent varier. Pour cela, l'ouverture 11 peut être à dimension variable (tel un diaphragme).

En variante, la paroi de séparation 10 est amovible de sorte qu'elle peut être remplacée par une nouvelle paroi 10 présentant une ouverture 11 ayant des dimensions différentes. Par exemple, une première paroi de séparation peut comprendre une ouverture 11 ayant un diamètre de 39 mm, tandis qu'une deuxième paroi peut présenter une ouverture 11 ayant un diamètre de 20 mm.

La paroi de séparation 10 est alors fixable de manière amovible et étanche dans l'enceinte, par exemple au moyen d'un joint 12 et le cas échéant par serrage de vis et écrous 13.

La paroi de séparation 10 peut être réalisée en acier inoxydable. En variante, afin de l'alléger, la paroi de séparation 10 peut comprendre une plaque en acier inoxydable 14 renfermée entre deux plaques de téflon 15.

Une membrane 40 recouvre l'ouverture 11.

Avantageusement, la membrane 40 est fixée de manière étanche sur l'ouverture 11, par exemple par l'intermédiaire d'un support en acier inoxydable 42 et d'un joint 41. Ici, le joint 41 est du type plat, tel qu'un joint plat en caoutchouc, un joint plat VITON^{®} ou un joint plat TEFLON^{®}.

Selon une forme de réalisation préférée, la membrane 40 est hémiperméable, c'est-à-dire qu'elle permet le passage des fluides (ici de l'air) en provenance de l'un des compartiments (ici, le compartiment supérieur 20), et limite, voire interdit, le passage des fluides en provenance de l'autre (ici, les vapeurs de substance volatile en provenance du compartiment inférieur 30). Plus précisément, et comme on le verra dans la suite de la description, la fonction de la membrane 40 est de limiter, voire bloquer, le passage des substances volatiles en provenance du compartiment inférieur 30, tout en autorisant les échanges d'oxygène et de gaz carbonique en provenance du compartiment supérieur 20 ou compartiment inférieur 30.

Par exemple, la membrane 40 peut être une membrane commerciale telle que celles employées généralement dans le domaine de la ventilation pour la filtration et la stérilisation de gaz.

De préférence, la membrane 40 est choisie de manière à avoir un diamètre de pores inférieur ou égal à 0.22 µm, afin de stériliser l'air la traversant du compartiment supérieur 20 vers le compartiment inférieur 30.

Typiquement, il peut s'agir d'une membrane Durapore^{®}, fournie par la société Millipore. Cette membrane est fabriquée à partir de polyfluorure de vinylidène et présente les caractéristiques constructeur suivantes : taille de pores = 0.1 µm, diamètre de la membrane compris entre 5 et 47 mm , épaisseur de la membrane = 125 µm, porosité (correspondant volume des pores dans la membrane) = 70%, débit d'air = 3 L/min/cm²), et une tortuosité pouvant être comprise entre 2 et 4.

Il peut également s'agir d'une membrane Emflon^{®} commercialisée par la société Pall Corporation. Cette membrane est fabriquée en polytétrafluoroéthylène (PFTE) et ses caractéristiques constructeur sont les suivantes : taille de pores = 0.02 µm, épaisseur de la membrane = 0,22 mm +/- 0,1 mm, porosité = 70%, débit d'air = 62,30 cm3 / min / cm² / psi, un diamètre de la membrane compris entre 5 et 100 mm et une tortuosité pouvant être comprise entre 2 et 4.

D'autres membranes en polymère poreux, métal poreux ou en céramique poreuse sont également utilisables, tant qu'elles sont hémiperméables.

On remarquera ici que la mise en oeuvre de ces membranes crée, au niveau de l'ouverture 11, une zone critique dans laquelle les particules de substance volatile s'accumulent sans pouvoir traverser vers le compartiment supérieur 20. On peut alors dire que cette accumulation forme une sorte de « barrière » renforçant la résistance constituée par la membrane 40, sans pour autant empêcher le passage de l'air provenant du compartiment supérieur 20.

La taille de la membrane 40 peut, le cas échéant, être ajustée en fonction du diamètre de l'ouverture 11 et du support prévu sur la paroi de séparation 10.

Le premier compartiment 20, dit ici supérieur, comprend une entrée et une sortie, s'étendant de préférence entre deux parois opposées 4a, 4b de l'enceinte 1, et adaptées pour permettre à un fluide gazeux de traverser le compartiment 20 d'une paroi 4a à l'autre 4b. Le débit du fluide gazeux peut être régulé de manière optionnelle par des vannes disposées respectivement au niveau de l'entrée 21 et de la sortie 22 du premier compartiment 20. Il peut par exemple être de l'ordre de 17,64 L.h⁻¹.

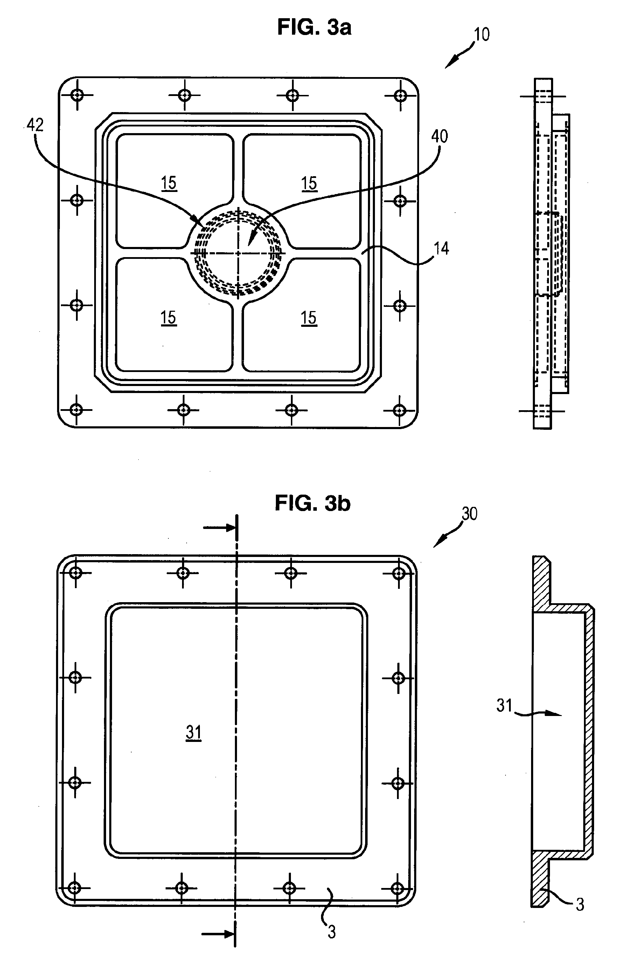

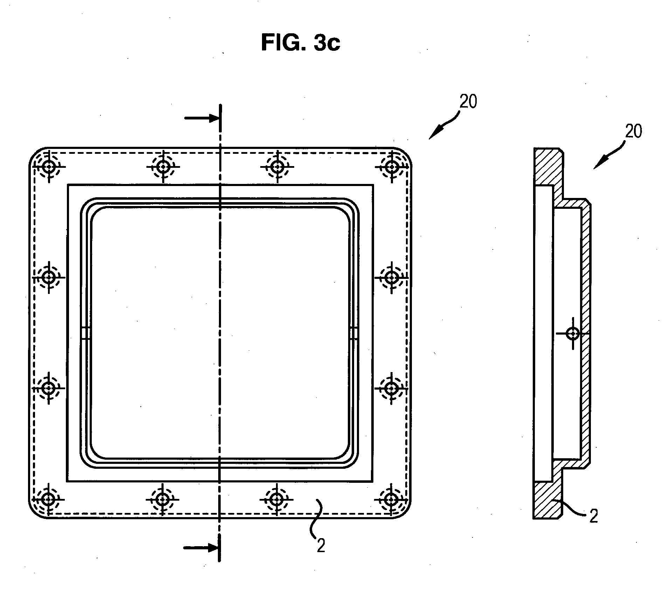

Le deuxième compartiment 30, dit ici inférieur, comporte une zone 31 adaptée pour recevoir une solution. Il peut s'agir notamment d'un récipient, etc. Sur la figure 3b, la zone 31 est un renfoncement réalisé dans la paroi inférieure 3 de l'enceinte 1.

Avantageusement, la zone 31 est en communication fluidique avec une vanne 32 adaptée pour introduire du fluide dans le compartiment inférieur 30, notamment du gaz. Le compartiment inférieur peut en outre comporter un septum 33, typiquement en PFTE ou en caoutchouc, en communication fluidique avec la zone 31 et adapté pour permettre le cas échéant un prélèvement de fluide (notamment pour une mesure de concentration, etc.). De la sorte, lorsque la vanne 32 et le septum 33 sont fermés, le compartiment inférieur 30 est étanche, de sorte que les seuls échanges avec le milieu extérieur au compartiment 30 ont lieu à travers la membrane 40 vers le compartiment supérieur 20.

Nous allons à présent décrire un procédé d'analyse des effets d'au moins un paramètre relatif à une substance volatile sur un système d'essai.

Dans ce qui suit, nous décrirons plus particulièrement le procédé dans son application à l'analyse de la toxicité de substances volatiles sur des cultures de cellules animales, végétales ou bactériennes. Par ailleurs, l'enceinte 1 choisie dans notre exemple mesure environ 5 cm de hauteur (entre les faces supérieure 2 et inférieure 3) pour une quinzaine de centimètres de largeur et de profondeur. Le rapport entre le volume du deuxième compartiment 30 sur le volume total de liquide est variable mais peut par exemple être compris entre 9 et 10, et le rapport entre le volume total du compartiment 30 et de la surface de transfert du fluide retenu par la zone 31 est de préférence de l'ordre de 3. Ceci n'est cependant pas limitatif.

Avant l'essai, il est préférable de porter l'enceinte 1 à une température fixe déterminée, par exemple 37°C +/- 1°C, de manière à la « thermostater ». Plus généralement, la température de l'enceinte dépend du type d'essai réalisé, de la substance volatile et du système d'essai, mais reste de préférence constante au cours de l'essai. On choisit alors un type d'ouverture 11 la paroi de séparation 10 correspondante, puis on la fixe de manière étanche dans l'enceinte de manière à former les deux compartiments 20, 30.

On fixe ensuite ou préalablement à la mise en place de la paroi de séparation 10 dans l'enceinte 1) de manière étanche, par exemple au moyen d'un joint 41, une membrane hémiperméable 40 sur la paroi de séparation 10.

Par ailleurs, afin de maintenir la température de l'enceinte 1 et des éléments qu'elle comprend constante lors des essais, l'enceinte est maintenue de préférence tout au long des essais à la température déterminée. Par exemple, l'enceinte est placée dans une étuve maintenue à 37°C.

L'enceinte 1 est alors prête à être utilisée.

On réalise une solution mère comprenant un fluide auquel on mélange la substance volatile à tester.

Le fluide peut être de l'eau, de préférence de l'eau ultra pure (par exemple l'eau ultra pure Millipore Q-Guard® 1) ou de l'eau distillée, le but étant de limiter toute contamination du fluide utilisé.

On mélange alors la substance volatile dans l'eau à une dose inférieure ou au plus égale à la limite de solubilité de ladite substance dans l'eau. Par exemple, on mélange à une concentration égale à 95% de la limite de solubilité de la substance dans l'eau.

Selon une forme de réalisation préférée, on homogénéise en outre la solution, par exemple dans un bain à ultrason, réglé à la même température que l'enceinte, ici 37°C. On peut alors stocker la solution mère jusqu'au moment de son utilisation dans un récipient étanche placé de préférence dans une étuve également thermostatée à 37°C.

Le gaz comprend de préférence 5% de dioxyde de carbone (CO₂) avec 95% d'humidité relative, de manière à simuler les conditions d'un incubateur mises en oeuvre pour la culture de cellule. Par exemple, on branche une bouteille 50 de gaz comprenant un mélange d'air avec 5% de CO₂, tel que le mélange « Cristal » d'Air Liquide, sur l'entrée 21 du compartiment supérieur 20 par l'intermédiaire d'un humidificateur 51 d'air adapté pour régler l'humidité relative de l'air à 95%.

L'air ainsi humidifié est alors introduit dans le compartiment supérieur 20, de préférence à un débit tel que l'écoulement dans le compartiment supérieur 20 est laminaire. Le débit d'air peut notamment être réglé au moyen des vannes situées à l'entrée 21 et à la sortie 22 du compartiment 20.

Avantageusement, l'air introduit dans le compartiment supérieur 20 est à la même température que l'enceinte 1, ici 37°C et à pression atmosphérique.

On introduit alors la solution mère dans la zone 31 du compartiment inférieur 30, de préférence à une température de 37°C, par l'intermédiaire de la vanne 32. Pour cela, on peut notamment utiliser une seringue 52 munie au niveau de son extrémité d'une connexion luer lock et d'une aiguille métallique.

Par ailleurs, le gaz présent dans le compartiment inférieur 30 peut être de l'air ambiant, de préférence à la même température que le reste du dispositif, ici 37°C.

Le système d'essai peut alors être placé dans le compartiment inférieur 30. Par exemple, dans le cas de cultures cellulaires, celles-ci sont introduites dans la zone 31 avec la solution mère.

Dans le cas d'animaux, ceux-ci sont placés dans le compartiment inférieur, par exemple en ouvrant l'enceinte 1 de manière à pouvoir y accéder. Pour cela, l'enceinte comporte une partie amovible, par exemple une fenêtre dans une paroi donnant accès au compartiment inférieur 30.

Bien entendu, l'ordre des étapes mentionnées ci-dessus n'est pas limitatif. Typiquement, il est possible d'introduire la solution mère contenant le composé volatile après avoir placé le système d'essai dans l'enceinte 1.

En variante, le compartiment supérieur 20, le compartiment inférieur 30 et la paroi 10 sont démontables.

Au bout d'un temps déterminé, fonction notamment du type de substance volatile testé, on retire alors le système d'essai du compartiment inférieur et on implémente des tests sur celui-ci afin d'évaluer l'au moins un paramètre de la substance volatile, typiquement sa toxicité.

La durée des essais dans l'enceinte peut être comprise entre 10 minutes et 48 heures.

Afin de mieux déterminer l'impact de la substance volatile sur le système d'essai, on détermine l'évolution de la concentration de la substance dans le temps.

Cette évolution est déterminée en particulier à partir de tout ou partie des caractéristiques suivantes : la surface de transfert de la solution mère dans le compartiment inférieur 30, le volume du compartiment inférieur 30, la hauteur de la solution mère dans le compartiment inférieur 30, le volume de solution mère et/ou le volume de gaz présent dans le compartiment inférieur 30, la température de la solution mère ou du gaz présent dans le compartiment inférieur 30, la pression dans le compartiment supérieur 20 (ici, la pression atmosphérique), la composition du gaz balayant le compartiment supérieur 20, le débit de gaz dans le compartiment supérieur 20, des propriétés de la substance volatile et des propriétés de la membrane 40.

Les propriétés de la substance volatile pouvant être prises en compte sont notamment son coefficient de partage, son coefficient de diffusion dans l'eau, son coefficient de diffusion dans l'air, sa viscosité en phase liquide, sa viscosité en phase vapeur, sa densité en phase liquide, ou encore sa densité en phase vapeur.

Néanmoins, la modélisation de l'évolution de la concentration dépend de la membrane 40 utilisée durant l'essai. Il faut donc en outre prendre en compte les propriétés de la membrane 40, qui peuvent notamment être sa porosité, la taille de ses pores et sa tortuosité.

Ces propriétés peuvent notamment être vérifiées préalablement à la mise en oeuvre du procédé d'analyse de l'invention, afin de prendre en compte des valeurs précises dans la modélisation de l'évolution de la concentration de la substance volatile dans le compartiment inférieur.

Par exemple, la porosité de la membrane 40 peut être mesurée à l'aide d'un porosimètre au mercure, sa tortuosité au moyen d'une expérience de perméation gazeuse et son épaisseur au microscope électronique à balayage.

Enfin, afin de valider la modélisation, il est possible d'effectuer des mesures à intervalles réguliers de la concentration de la substance volatile dans la solution mère. Pour cela, on peut notamment prélever un échantillon (typiquement 1 µL, afin de ne pas modifier l'équilibre thermodynamique dans le compartiment inférieur 30) de solution mère à intervalles réguliers et en mesurer la concentration par chromatographie en phase gazeuse, puis comparer l'évolution de la concentration mesurée avec les résultats de la modélisation. En effet, lorsque le système est à l'équilibre dans le compartiment inférieur 30, la concentration de la substance volatile est constante dans la solution mère. Par conséquent, une diminution de la concentration de la solution mère implique un transfert d'une partie de la substance volatile à travers la membrane 40.

## Revendications

1. Dispositif d'analyse des effets d'au moins un paramètre relatif à une substance volatile sur un système d'essai vivant, **caractérisé en ce qu'**il comprend :
- une enceinte (1) composée d'un premier compartiment (20) comprenant une entrée (21) et une sortie (22) de gaz, et d'un deuxième compartiment (30) dans lequel sont disposés le système d'essai et la substance volatile,
- les premier (20) et deuxième (30) compartiments communiquant l'un avec l'autre par l'intermédiaire d'une ouverture (11) munie d'une membrane hémiperméable (40) adaptée pour limiter de manière déterminée un flux de substance volatile du deuxième compartiment (30) vers le premier compartiment (20), tout en permettant un flux de gaz du premier compartiment (20) vers le deuxième compartiment (30), et
- le deuxième compartiment (30) étant hermétique de sorte que les seuls échanges avec un milieu extérieur aient lieu vers le premier compartiment (20), à travers la membrane (40).

2. Dispositif d'analyse selon la revendication 1, **caractérisé en ce que** le paramètre est la toxicité de la substance volatile.

3. Dispositif d'analyse selon l'une des revendications 1 et 2, **caractérisé en ce que** le gaz comprend 5% de dioxyde de carbone et 95% d'humidité relative.

4. Dispositif d'analyse selon l'une des revendications 1 à 3, **caractérisé en ce que** la substance volatile est mélangée à un fluide de manière à obtenir une solution mère, et **en ce que** le dispositif comprend en outre une zone (31) adaptée pour recevoir ladite solution mère.

5. Dispositif d'analyse selon la revendication 4, **caractérisé en ce que** le fluide utilisé dans l'obtention de la solution mère est de l'eau distillée ou de l'eau extra pure.

6. Dispositif d'analyse selon l'une des revendications 4 ou 5, **caractérisé en ce que** la solution mère est introduite par l'intermédiaire d'une vanne (32).

7. Dispositif d'analyse selon l'une des revendications 1 à 6, **caractérisé en ce que** la membrane (40) est fixée sur l'ouverture (11) par l'intermédiaire d'un joint (41).

8. Procédé d'analyse des effets d'au moins un paramètre relatif à une substance volatile sur un système d'essai vivant à l'aide d'un dispositif selon l'une des revendications 1 à 7, **caractérisé en ce qu'**il comprend les étapes suivantes :
- mélanger la substance volatile avec un fluide de manière à obtenir une solution mère homogène,
- placer la membrane (40) sur l'ouverture (11) de manière étanche,
- introduire la solution mère dans le deuxième compartiment (30) et fermer le deuxième compartiment (30), de sorte que les seuls échanges avec un milieu extérieur aient lieu vers le premier compartiment (20), à travers la membrane (40),
- faire passer un flux gazeux dans le premier compartiment (20) pendant un temps déterminé,
- déterminer l'évolution dans le temps de la concentration de la substance volatile dans le deuxième compartiment (30), et
- à partir de l'évolution dans le temps de la concentration de la substance volatile, analyser le système d'essai et en déduire le paramètre relatif à la substance volatile.

9. Procédé selon la revendication 8, comprenant en outre une étape au cours de laquelle l'enceinte (1) est placée à une température déterminée préalablement à l'introduction de la solution mère.

10. Procédé selon la revendication 9, dans lequel la solution mère et le flux gazeux balayant le premier compartiment (20) sont introduits à la température déterminée de l'enceinte (1).

11. Procédé selon la revendication 10, dans lequel la température déterminée est d'environ 37°C +/- 1°C.

12. Procédé selon l'une des revendications 8 à 11, dans lequel l'évolution dans le temps de la concentration de la substance volatile est déterminée en fonction de l'un au moins des paramètres suivants :
- la surface de transfert de la solution mère dans le deuxième compartiment (30),
- le volume du deuxième compartiment,
- la hauteur de la solution mère dans le deuxième compartiment (30),
- le volume de solution mère et/ou le volume de gaz présent dans le deuxième compartiment (30),
- la température de la solution mère ou du gaz présent dans le deuxième compartiment (30),
- la pression dans le premier compartiment (20) et/ou dans le deuxième compartiment (30),
- la composition du gaz balayant le premier compartiment (20),
- le débit de gaz dans le premier compartiment (20),
- des propriétés de la substance volatile et,
- des propriétés de la membrane (40).

13. Procédé selon la revendication 12, dans lequel les propriétés de la substance volatile comprennent l'un au moins des éléments du groupe suivant : son coefficient de partage, son coefficient de diffusion dans l'eau, son coefficient de diffusion dans l'air, sa viscosité en phase liquide, sa viscosité en phase vapeur, sa densité en phase liquide, et sa densité en phase vapeur.

14. Procédé selon l'une des revendications 12 ou 13, dans lequel les propriétés de la membrane (40) comprennent l'un au moins de éléments du groupe suivant : sa porosité, la taille de ses pores, et sa tortuosité.

15. Procédé selon l'une des revendications 8 à 14, comprenant en outre une étape préalable d'homogénéisation de la solution mère.

## Patentansprüche

1. Vorrichtung zur Untersuchung der Auswirkungen wenigstens eines Parameters, der zu einer flüchtigen Substanz gehört, auf ein lebendes Versuchssystem, **dadurch gekennzeichnet, dass** sie Folgendes umfasst:
- einen umfassten Raum (1), der aus einem ersten Abteil (20), das einen Einlass (21) und einen Auslass (22) für Gas umfasst, und einem zweiten Abteil (30), in dem das Versuchssystem und die flüchtige Substanz angeordnet sind, zusammengesetzt ist,
- wobei das erste (20) und zweite (30) Abteil untereinander über eine Öffnung (11) im Austausch stehen, die mit einer semipermeablen Membran (40) ausgestattet ist, die dafür eingerichtet ist, auf definierte Weise einen Fluss der flüchtigen Substanz vom zweiten Abteil (30) in Richtung des ersten Abteils (20) zu begrenzen und dabei gleichzeitig einen Gasfluss vom ersten Abteil (20) in Richtung des zweiten Abteils (30) zuzulassen, und
- wobei das zweite Abteil (30) hermetisch dicht ist, derart, dass der einzige Austausch mit einer äußeren Umgebung mit dem ersten Abteil (20) stattfindet, und dies durch die Membran (40) hindurch.

2. Untersuchungsvorrichtung nach Anspruch 1, **dadurch gekennzeichnet, dass** der Parameter die Toxizität der flüchtigen Substanz ist.

3. Untersuchungsvorrichtung nach einem der Ansprüche 1 und 2, **dadurch gekennzeichnet, dass** das Gas 5% Kohlendioxid und 95% relative Feuchtigkeit enthält.

4. Untersuchungsvorrichtung nach einem der Ansprüche 1 bis 3, **dadurch gekennzeichnet, dass** die flüchtige Substanz auf solche Art mit einem Fluid gemischt wird, dass eine Mutterlösung erhalten wird, und dadurch, dass die Vorrichtung außerdem einen Bereich (31) umfasst, der dafür eingerichtet ist, die Mutterlösung aufzunehmen.

5. Untersuchungsvorrichtung nach Anspruch 4, **dadurch gekennzeichnet, dass** das Fluid, das bei der Gewinnung der Mutterlösung verwendet wird, destilliertes Wasser oder extra reines Wasser ist.

6. Untersuchungsvorrichtung nach einem der Ansprüche 4 oder 5, **dadurch gekennzeichnet, dass** die Mutterlösung mittels eines Ventils (32) eingebracht wird.

7. Untersuchungsvorrichtung nach einem der Ansprüche 1 bis 6, **dadurch gekennzeichnet, dass** die Membran (40) mittels einer Dichtung (41) an der Öffnung (11) befestigt ist.

8. Verfahren zur Untersuchung der Auswirkungen wenigstens eines Parameters, der zu einer flüchtigen Substanz gehört, auf ein lebendes Versuchssystem mittels einer Vorrichtung nach einem der Ansprüche 1 bis 7, **dadurch gekennzeichnet, dass** es die folgenden Schritte umfasst:
- Mischen der flüchtigen Substanz mit einem Fluid, und dies derart, dass eine homogene Mutterlösung erhalten wird,
- Anbringen der Membran (40) an der Öffnung (11) auf abdichtende Weise,
- Einbringen der Mutterlösung in das zweite Abteil (30) und Verschließen des zweiten Abteils (30), derart, dass der einzige Austausch mit einer äußeren Umgebung mit dem ersten Abteils (20) stattfindet, und dies durch die Membran (40) hindurch,
- Leiten eines Gasstroms durch das erste Abteil (20) während einer definierten Zeitspanne,
- Festlegen des zeitlichen Verlaufs der Konzentration der flüchtigen Substanz im zweiten Abteil (30), und
- Untersuchen des Versuchssystems, ausgehend vom zeitlichen Verlauf der Konzentration der flüchtigen Substanz, und darauf basierend, Ableiten des Parameters, der zu der flüchtigen Substanz gehört.

9. Verfahren nach Anspruch 8, das außerdem einen Schritt umfasst, während dessen der umfasst Raum (1) auf eine definierte Temperatur gebracht wird, und dies bevor die Mutterlösung eingebracht wird.

10. Verfahren nach Anspruch 9, bei dem die Mutterlösung und der Gasfluss, der durch das erste Abteil (20) strömt, mit der für den umfassten Raum (1) definierten Temperatur eingebracht werden.

11. Verfahren nach Anspruch 10, bei dem die definierte Temperatur in etwa 37 °C +/- 1 °C beträgt.

12. Verfahren nach einem der Ansprüche 8 bis 11, bei dem der zeitliche Verlauf der Konzentration der flüchtigen Substanz in Abhängigkeit von wenigstens einem der folgenden Parameter festgelegt wird:
- der Übertragungsoberfläche der Mutterlösung im zweiten Abteil (30),
- dem Volumen des zweiten Abteils,
- der Höhe der Mutterlösung im zweiten Abteil (30),
- dem Volumen der Mutterlösung und/oder dem Gasvolumen, das im zweiten Abteil (30) vorhanden ist,
- der Temperatur der Mutterlösung oder des Gases, die im zweiten Abteil (30) vorhanden sind,
- dem Druck im ersten Abteil (20) und/oder im zweiten Abteil (30),
- der Zusammensetzung des Gases, das durch das erste Abteil (20) strömt,
- der Gasdurchflussmenge im ersten Abteil (20),
- Eigenschaften der flüchtigen Substanz, und
- Eigenschaften der Membran (40).

13. Verfahren nach Anspruch 12, bei dem die Eigenschaften der flüchtigen Substanz wenigstens eines der Elemente aus der folgenden Gruppe umfassen:
ihrem Verteilungskoeffizienten, ihrem Diffusionskoeffizienten im Wasser, ihrem Diffusionskoeffizienten im Gas, ihrer Viskosität in der flüssigen Phase, ihrer Viskosität in der Dampfphase, ihrer Dichte in der flüssigen Phase und ihrer Dichte in der Dampfphase.

14. Verfahren nach einem der Ansprüche 12 oder 13, bei dem die Eigenschaften der Membran (40) wenigstens eines der Elemente aus der folgenden Gruppe umfassen: ihrer Porosität, der Größe ihrer Poren und ihrer Tortuosität.

15. Verfahren nach einem der Ansprüche 8 bis 14, das außerdem einen vorangehenden Schritt zur Homogenisierung der Mutterlösung umfasst.

## Claims

1. Device for analysing the effects of at least one parameter related to a volatile substance on a living test system, **characterised in that** it comprises:
- an enclosure (1) composed of a first compartment (20) comprising a gas inlet (21) and a gas outlet (22), and a second compartment (30) in which the test system and the volatile substance are contained,
- the first (20) and second (30) compartments communicate with each other through an opening (11) fitted with a semi-permeable membrane (40) adapted to limit a flow of volatile substance from the second compartment (30) to the first compartment (20) in a determined manner, while allowing a gas flow from the first compartment (20) to the second compartment, (30), and
- the second compartment (30) being hermetically sealed such that the only exchanges with an outside medium take place towards the first compartment (20), through the membrane (40).

2. Analysis device according to claim 1, **characterised in that** the parameter is the toxicity of the volatile substance.

3. Analysis device according to one of claims 1 and 2, **characterised in that** the gas comprises 5% carbon dioxide and 95% relative humidity.

4. Analysis device according to one of claims 1 to 3, **characterised in that** the volatile substance is mixed with a fluid so as to obtain a mother solution, and **in that** the device also comprises a zone (31) adapted to contain said mother solution.

5. Analysis device according to claim 4, **characterised in that** the fluid used for obtaining the mother solution is distilled water or extra pure water.

6. Analysis device according to one of claims 4 or 5, **characterised in that** the mother solution is introduced through a valve (32).

7. Analysis device according to one of claims 1 to 6, **characterised in that** the membrane (40) is fixed to the opening (11) through a gasket (41).

8. Method for analysing the effects of at least one parameter related to a volatile substance on a living test system using a device according to one of claims 1 to 7, **characterised in that** it comprises the following steps:
- mix the volatile substance with a fluid so as to obtain an homogeneous mother solution,
- place the membrane (40) on the opening (11) in a sealed manner,
- add the mother solution into the second compartment (30) and close the second compartment (30), so that the only exchanges with an outside medium take place towards the first compartment (20), through the membrane (40),
- transfer a gas flow into the first compartment (20) for a determined period,
- determine the variation in the concentration of the volatile substance in the second compartment (30), and
- starting from the variation of the concentration of the volatile substance with time, analyse the test system and use the analysis to determine the parameter for the volatile substance.

9. Method according to claim 8, also comprising a step during which the enclosure (1) is brought to a determined temperature before the mother solution is added.

10. Method according to claim 9, in which the mother solution and the gas flow scavenging the first compartment (20) are added at the determined temperature of the enclosure (1).

11. Method according to claim 10, in which the determined temperature is about 37°C +/- 1°C.

12. Method according to one of claims 8 to 11, in which the variation in the concentration of the volatile substance in time is determined as a function of at least one of the following parameters:
- a transfer area of the mother solution into the second compartment (30),
- a volume of the second compartment,
- a depth of the mother solution in the second compartment (30),
- a volume of the mother solution and/or the gas volume present in the second compartment (30),
- a temperature of the mother solution or the gas present in the second compartment (30),
- a pressure in the first compartment (20) and/or the second compartment (30),
- a composition of the gas scavenging the first compartment (20),
- a gas flow in the first compartment (20),
- properties of the volatile substance, and
- properties of the membrane (40).

13. Method according to claim 12, in which the properties of the volatile substance comprise at least one of the elements in the following group: its sharing coefficient, its coefficient of diffusion in water, its coefficient of diffusion in air, its viscosity in the liquid phase, its viscosity in the vapour phase, its density in the liquid phase and its density in the vapour phase.

14. Method according to one of claims 12 or 13, in which properties of the membrane (40) comprise at least one of the elements in the following group: its porosity, the pore size and its tortuosity.

15. Method according to one of claims 8 to 14, also including a preliminary step to homogenise the mother solution.
